# EUROPEAN PATENT APPLICATION

(11) **EP 4 830 699 A1**
(43) Date of publication of application: **28.10.2026**
(21) Application number: 26166589.7
(22) Date of filing: 21.03.2026
(51) Int. Cl.: A61K 9/06, A61K 9/00, A61K 9/127, A61K 9/1277, A61K 31/045, A61K 31/415, A61K 31/445, A61K 31/635, A61K 31/5415, A61K 45/06, A61K 47/10, A61K 47/14, A61P 29/00

(54) **USE OF PHARMACEUTICAL COMPOSITION IN THE PREPARATION OF DRUG FOR ENHANCING ANALGESIC EFFECT**

(30) Priority: 24.04.2025 CN 202510522268
(71) Applicant: Brightintel Biotech Pharmaceutical Co., Ltd., Suzhou, Jiangsu 215000 (CN)
(72) Inventor: LI, Fengquan, Jiangsu, 215000 (CN); LI, Qing, Jiangsu, 215000 (CN); YE, Qiongru, Jiangsu, 215000 (CN)
(74) Representative: Purdylucey Intellectual Property

(57) **Abstract**

The present disclosure belongs to the technical field of pharmaceutical formulations, and in particular relates to use of a pharmaceutical composition in the preparation of a drug for enhancing analgesic effect. The pharmaceutical composition of the present disclosure includes an analgesic drug and a terpene drug. The analgesic drug includes at least one of a local anesthetic and a nonsteroidal anti-inflammatory drug; and the terpene drug includes at least one of menthol, camphor, linalool, and eucalyptol. The analgesic drug and the terpene drug of the present disclosure have a synergistic effect.

## Description

### TECHNICAL FIELD

The present disclosure belongs to the technical field of pharmaceutical formulations, and in particular relates to use of a pharmaceutical composition in the preparation of a drug for enhancing analgesic effect.

### BACKGROUND

At present, one or more of opioid analgesics, nonsteroidal analgesics, and local anesthetics are mainly used in clinical practice for postoperative analgesia. Compared with other local anesthetics, local anesthetics such as bupivacaine hydrochloride injection and ropivacaine hydrochloride injection, etc., have a long duration of action, but the analgesic effect of a single administration is only 6-8 hours, which cannot meet the treatment cycle for postoperative pain and is difficult to relieve the postoperative suffering of patients for a long time.

Existing long-acting postoperative analgesic drugs typically achieve prolonged analgesic effects by extending the release of bupivacaine. There are four common long-acting sustained-release formulations of bupivacaine: one is an implant (Xaracoll from Innocoll), one is liposome (Exparel from Pacira), and the other two are gel formulations (Posimir from Durect, and Zynrelef from Heron). In order to achieve better therapeutic effects, the aforementioned four long-acting sustained-release formulations require high drug-loading capacity. Approaches to increase drug-loading capacity usually involve increasing the volume of solvent. However, this often necessitates injecting more than 20 mL of solution near a surgical incision site, potentially causing local swelling and more adverse effects. Moreover, among the aforementioned four long-acting postoperative analgesic drugs, Zynrelef can only provide an analgesic duration of 72 hours, while the other three analgesic drugs have an analgesic duration of 24-48 hours, which cannot well meet the clinical demand for longer-acting analgesic drugs.

Therefore, it is of great significance to provide use of a pharmaceutical composition that can enhance the analgesic effect without changing the releasing rate of analgesic drugs and has a long-acting analgesic effect in the preparation of a drug for enhancing analgesic effect.

### SUMMARY

The present disclosure aims to solve one or more of the above-mentioned technical problems existing in the existing technology, and at least provide a beneficial option or create conditions. Specifically, the present disclosure provides use of a pharmaceutical composition in the preparation of a drug for enhancing analgesic effect, where the pharmaceutical composition can enhance the analgesic effect without changing the releasing rate of an analgesic drug, prolong the analgesic duration of a drug, and has a longer-acting analgesic effect.

The inventive concept of the present disclosure is to provide use of a pharmaceutical composition in the preparation of a drug for enhancing analgesic effect. The pharmaceutical composition includes an analgesic drug and a terpene drug. The analgesic drug includes at least one of a local anesthetic and a nonsteroidal anti-inflammatory drug, and the terpene drug includes at least one of menthol, camphor, linalool, and eucalyptol. The analgesic drug and the terpene drug of the present disclosure have a synergistic effect. Without changing the release characteristics of the analgesic drug, the terpene drug can increase the drug-loading capacity of the analgesic drug, achieving the purpose of enhancing and prolonging the analgesic effect. The analgesic duration in Bama pigs can be extended to 120 hours, demonstrating a significantly superior long-acting analgesic effect compared to the existing marketed drug Zynrelef. Furthermore, an increase in the drug-loading capacity of the analgesic drug allows for a reduced amount of solvent required, enabling a smaller injection volume and improving the compliance and safety of patients.

Therefore, a first aspect of the present disclosure provides use of a pharmaceutical composition in the preparation of a drug for enhancing analgesic effect.

Specifically, the pharmaceutical composition includes an analgesic drug and a terpene drug. The analgesic drug includes at least one of a local anesthetic and a nonsteroidal anti-inflammatory drug; and the terpene drug includes at least one of menthol, camphor, linalool and eucalyptol.

The terpene drug, particularly represented by menthol, exert its analgesic effect by stimulating cold receptors in the skin to produce a cooling sensation that masks pain perception. The analgesic effect of menthol is dose-dependent. When administered through the skin, it provides a surface analgesic effect. At higher doses, menthol can cause discomfort to wounds, whereas at low doses, menthol cannot exert an analgesic effect. These constraints limit the use of the terpene drug in injection scenarios for acute pain. Especially in the field of postoperative analgesia, there has been no clinical use of the terpene drug for long-acting postoperative analgesia. After long-term experimental researches, the present disclosure has broken through the limitation. Due to the synergistic effect of the terpene drug and the analgesic drug, the therapeutic effect of the analgesic drug can be improved without changing the release characteristics of the analgesic drug. By using the terpene drug to increase the drug-loading capacity of the analgesic drug, the dosage of the analgesic drug required can be reduced, achieving the purpose of enhancing and prolonging the drug efficacy, improving patient compliance, and reducing adverse reactions of the drug.

Preferably, the menthol is L-menthol.

Preferably, the local anesthetic includes at least one of bupivacaine and ropivacaine.

Preferably, the nonsteroidal anti-inflammatory drug includes at least one of meloxicam and celecoxib.

Preferably, the pharmaceutical composition further includes at least one of a delivery carrier and a solvent.

Specifically, the pharmaceutical composition is a sustained-release formulation, and the delivery carrier has a sustained-release effect.

Preferably, the delivery carrier includes at least one of a saccharide and an esterified form thereof, a phospholipid, a polyester, and an oil.

Preferably, the solvent includes alcohols, dimethyl sulfoxide, ethyl lactate, glycerol formal, N-methylpyrrolidone, glycerol triacetate (triacetin), benzyl benzoate, dichloromethane, water, and glycerol trioctanoate.

More preferably, the alcohols include at least one of methanol, ethanol, n-propanol, isopropanol, n-butanol, isobutanol, tert-butanol, ethylene glycol, propylene glycol, glycerol, benzyl alcohol, phenethyl alcohol, and polyethylene glycol.

Preferably, the saccharide and an esterified form thereof include at least one of sucrose, chitosan, sucrose acetate isobutyrate, sucrose octaacetate, and sucrose monoacetate monoisobutyrate.

Preferably, the phospholipid includes at least one of a natural phospholipid and a synthetic phospholipid.

Preferably, the natural phospholipid includes at least one of soybean phosphatidylcholine, egg yolk phospholipid, rapeseed phospholipid, and sunflower phospholipid.

Preferably, the synthetic phospholipid includes at least one of dierucoyl phosphatidylcholine (DEPC), dioleoyl phosphatidylcholine, palmitoyl oleoyl phosphatidylcholine, distearoyl phosphatidylcholine, dipalmitoyl phosphatidylglycerol (DPPG), and distearoyl phosphatidylglycerol.

Preferably, the polyester includes at least one of polylactic acid (PLA), poly(lactic-co-glycolic acid) (PLGA), polyglycolic acid (PGA), polydioxanone (PDO), polycaprolactone (PCL), poly(L-lactide-co-caprolactone) (PLCL), and poly(orthoester).

Preferably, the poly(orthoester) is selected from a non-thermoplastic poly(orthoester) having the following structure: 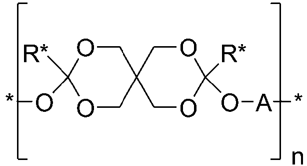
where R* is selected from any one of methyl, ethyl, propyl, or butyl; n is an integer from 5 to 400; and A is selected from any one of R¹ or R³;
R¹ is 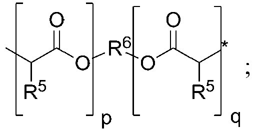 R⁵ is selected from any one of H or C₁₋₄ alkyl; R⁶ is 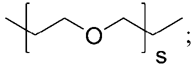 s is an integer from 0 to 10; and p and q are each independently selected from an integer from 1 to 20;
R³ is 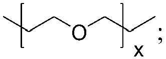 and x is an integer from 1 to 5.

Preferably, the oil includes at least one of castor oil, soybean oil, olive oil, palm oil, and sesame oil.

Preferably, the pharmaceutical composition further includes a delivery carrier and a solvent; and in parts by weight, the pharmaceutical composition includes 0.01-22 parts of the analgesic drug, greater than or equal to 0.01 parts and less than 5 parts of the terpene drug, 1-95 parts of the solvent, and 0.5-65 parts of the delivery carrier.

More preferably, in parts by weight, the pharmaceutical composition includes 0.01-20 parts of the analgesic drug, 0.01-4.5 parts of the terpene drug, 2-90 parts of the solvent, and 0.5-60 parts of the delivery carrier.

Preferably, a mass ratio of the local anesthetic to the terpene drug is 1 to 50: 1.

More preferably, the mass ratio of the local anesthetic to the terpene drug is 2 to 50: 1.

Preferably, the pharmaceutical composition exhibits a time to peak plasma concentration of 0.5-24 hours after subcutaneous injection into the back of rats.

More preferably, the pharmaceutical composition exhibits a time to peak plasma concentration of 0.5-12 hours after subcutaneous injection into the back of rats.

Still more preferably, the pharmaceutical composition exhibits a time to peak plasma concentration of 0.5-2 hours after subcutaneous injection into the back of rats.

Preferably, a dosage form of the pharmaceutical composition includes any one of a gel formulation or a liposome.

Preferably, the administration forms of the gel formulation and the liposome include an injection; the gel formulation and the liposome are administered via injection.

Preferably, the liposome includes any one of a multilamellar vesicle or a multivesicular liposome.

Preferably, the gel formulation is an in-situ gel formulation.

Preferably, when the dosage form of the pharmaceutical composition is liposome, in parts by weight, the pharmaceutical composition includes 0.01-22 parts of the analgesic drug, greater than or equal to 0.01 parts and less than 5 parts of the terpene drug, 0.5-33 parts of the phospholipid, and 60-95 parts of the solvent.

More preferably, when the dosage form of the pharmaceutical composition is liposome, in parts by weight, the pharmaceutical composition includes 0.01-20 parts of the analgesic drug, 0.01-4.5 parts of the terpene drug, 0.5-30 parts of the phospholipid, and 60-90 parts of the solvent.

Preferably, when the dosage form of the pharmaceutical composition is an in-situ gel formulation, in parts by weight, the pharmaceutical composition includes 0.01-22 parts of the analgesic drug, greater than or equal to 0.01 parts and less than 5 parts of the terpene drug, 2-60 parts of the saccharide and an esterified form thereof or poly(orthoester), and 2-60 parts of the solvent.

More preferably, when the dosage form of the pharmaceutical composition is an in-situ gel formulation, in parts by weight, the pharmaceutical composition includes 0.01-20 parts of the analgesic drug, 0.01-4.5 parts of the terpene drug, 3-50 parts of the saccharide and an esterified form thereof or poly(orthoester), and 2-50 parts of the solvent.

Preferably, a preparation method of the pharmaceutical composition includes the step of:
mixing raw material components to prepare the pharmaceutical composition.

Preferably, when the dosage form of the pharmaceutical composition is the liposome, the preparation method of the pharmaceutical composition includes the following steps:
dissolving the phospholipid in the solvent to obtain a solution 1; dissolving the analgesic drug and the terpene drug in water to obtain a solution 2; mixing the solution 1 and the solution 2 to form a primary emulsion; shearing the primary emulsion to obtain a multiple emulsion; and obtaining the pharmaceutical composition in the dosage form of a liposome by replacement concentration.

Preferably, when the dosage form of the pharmaceutical composition is the in-situ gel formulation, the preparation method of the pharmaceutical composition includes the following steps:
mixing the raw material components, heating and stirring until dissolved, followed by sterile filtration or moist-heat sterilization to obtain the pharmaceutical composition in the dosage form of the in-situ gel formulation.

Compared to the prior art, the technical scheme provided by the present disclosure has the following the beneficial effects.
(1) The analgesic drug and the terpene drug of the present disclosure have a synergistic effect. Without changing the release of the analgesic drug in the blood concentration in vivo, the purpose of enhancing and prolonging the analgesic effect is unexpectedly achieved by increasing the drug-loading capacity of the analgesic drug through the terpene drug. In the trials of postoperative analgesic effect on Bama pigs, the pharmaceutical composition still exhibits significant analgesic effect at 120 hours postoperatively. That is, the postoperative analgesic duration in Bama pigs can be extended to 120 hours, and the long-acting analgesic effect of the pharmaceutical composition is significantly superior to the existing marketed drug Zynrelef. In addition, the pharmaceutical composition of the present disclosure exhibits rapid onset of analgesia. In SD rats, a pronounced analgesic effect is observed as early as 0.5 hours after administration, and the maximum analgesic threshold, comparable to the preoperative level, is reached 2 hours after administration. In Bama pigs, the analgesic threshold comparable to the preoperative level can be achieved 1 hour after administration.
(2) Through long-term experimental researches, the present disclosure overcomes the limitations that the terpene drug exhibits poor analgesic effects at low usage amount and cause wound discomfort such as redness and swelling at high usage amount. Through the synergistic effect of the terpene drug and the analgesic drug, the terpene drug increases the drug-loading capacity of the analgesic drug, reduces the usage amount of the analgesic drug, and thus lowers the volume of solvent needed. This enables a smaller injection volume, thereby improving patient compliance, reducing adverse reactions of the drug, and simultaneously enhancing and prolonging analgesic effect.
(3) The sucrose acetate isobutyrate (SAIB) in the delivery carrier of the present disclosure also functions as a solubilizer, which can further increase the drug-loading capacity, and thus improving the analgesic effect.
(4) The preparation method of the pharmaceutical composition of the present disclosure is simple, easy to implement, cost-effective, and suitable for large-scale industrial production.

### BRIEF DESCRIPTION OF DRAWINGS

Fig. 1 shows plasma concentration curves of bupivacaine in rats after administration of the pharmaceutical compositions of Examples 2-3, Comparative Examples 1, 3, and 5 of the present disclosure;
Fig. 2 shows plasma concentration curves of menthol in rats after administration of the pharmaceutical compositions of Examples 2-3, Comparative Examples 2, 4, and 5 of the present disclosure;
Fig. 3 shows an analgesic effect of the pharmaceutical compositions of Example 2 and Comparative Examples 3-4 of the present disclosure in rats after administration;
Fig. 4 shows an analgesic effect of the pharmaceutical compositions of Examples 1-3, Example 8, and Comparative Example 6 of the present disclosure in rats after administration; and
Fig. 5 shows an analgesic effect of the pharmaceutical compositions of Examples 4-5 and Comparative Example 7 of the present disclosure as well as the existing analgesic drug Zynrelef of Comparative Example 8 in Bama miniature pigs after administration.

### DETAILED DESCRIPTION

To enable those having ordinary skills in the art to understand the technical scheme of the present disclosure more clearly and thoroughly, the following examples are provided for illustration. It should be noted that the following examples do not constitute a limitation on the protection scope claimed by the present disclosure.

Unless otherwise specified, all raw materials, reagents or apparatuses used in the following examples are commercially available from conventional sources, or can be prepared by known methods in the prior art.

The poly(orthoester) in the Examples and Comparative Examples of the present disclosure is tri(ethylene glycol) poly(orthoester).

The raw material components and amounts of the pharmaceutical compositions in Examples 1-9 and Comparative Examples 1-7 of the present disclosure are shown in Table 1, where "-" in Table 1 indicates that the corresponding component is not added.

**Table 1: Raw material components and amounts (in parts by weight) of the pharmaceutical compositions in Examples 1-9 and Comparative Examples 1-7.**

| Groups | Analgesic drugs and Terpene drugs | | | Solvents | | Delivery carriers | | | | |
|---|---|---|---|---|---|---|---|---|---|---|
| | Bupi vacai ne | L-me nthol | Celec oxib | Glycerol formal or glycerol trioctanoate or benzyl alcohol | Wat er | SAIB | Poly(or thoester ) | Castor oil | DEP C | DPP G |
| Example 1 | 1 | 0.05 | - | Glycerol formal/3 | - | 10 | - | - | - | - |
| Example 2 | 1 | 0.05 | - | Glycerol formal/3 | - | - | 10 | - | - | - |
| Example 3 | 1 | 0.05 | - | Glycerol formal/3 | - | - | - | 10 | - | - |
| Example 4 | 1 | 0.05 | 0.03 | Glycerol formal/3 | - | 10 | - | - | - | - |
| Example 5 | 0.5 | 0.05 | 0.015 | Glycerol formal/3 | - | 10 | - | - | - | - |
| Example 6 | 1 | 1 | - | Glycerol formal/3 | - | - | 10 | - | - | - |
| Example 7 | 1 | 0.5 | - | Glycerol formal/3 | - | - | 10 | - | - | - |
| Example 8 | 1 | 0.05 | - | Glycerol trioctanoate/ 0.15 | 75 | - | - | - | 0.6 | 0.07 |
| Example 9 | 20 | 4.5 | - | Benzyl alcohol/25 | - | 50.5 | - | - | - | - |
| Comparative example 1 | 1.05 | - | - | Glycerol formal/10 | - | - | - | - | - | - |
| Comparative Example 2 | - | 1.05 | - | Glycerol formal/10 | - | - | - | - | - | - |
| Comparative Example 3 | 1.05 | - | - | Glycerol formal/3 | - | 10 | - | - | - | - |
| Comparative Example 4 | - | 1.05 | - | Glycerol formal/3 | - | 10 | - | - | - | - |
| Comparative Example 5 | 1 | 0.05 | - | Glycerol formal/10 | - | - | - | - | - | - |
| Comparative Example 6 | 1 | 5 | - | Glycerol formal/3 | - | - | 10 | - | - | - |
| Comparative Example 7 | 1 | - | 0.03 | Glycerol formal/3 | - | 10 | - | - | - | - |

### Example 1

Use of a pharmaceutical composition in the preparation of a drug for enhancing analgesic effect is provided. Raw material components and amount of the pharmaceutical composition are shown in Table 1.

A preparation method of the pharmaceutical composition in Example 1 included the following steps:
mixing bupivacaine, L-menthol, glycerol formal and SAIB; heating and stirring at 65°C until dissolved; and then performing sterile filtration to obtain the pharmaceutical composition (as an in-situ gel formulation).

### Example 2

Use of a pharmaceutical composition in the preparation of a drug for enhancing analgesic effect is provided. Raw material components and amount of the pharmaceutical composition are shown in Table 1.

A preparation method of the pharmaceutical composition in Example 2 included the following steps:
mixing bupivacaine, L-menthol, glycerol formal and poly(orthoester); heating and stirring at 65°C until dissolved; and then performing sterile filtration to obtain the pharmaceutical composition (as an in-situ gel formulation).

### Example 3

Use of a pharmaceutical composition in the preparation of a drug for enhancing analgesic effect is provided. Raw material components and amount of the pharmaceutical composition are shown in Table 1.

A preparation method of the pharmaceutical composition in Example 3 included the following step:
mixing bupivacaine, L-menthol, glycerol formal and castor oil to obtain the pharmaceutical composition.

### Example 4

Use of a pharmaceutical composition in the preparation of a drug for enhancing analgesic effect is provided. Raw material components and amount of the pharmaceutical composition are shown in Table 1.

A preparation method of the pharmaceutical composition in Example 4 included the following steps:
mixing bupivacaine, L-menthol, celecoxib, glycerol formal and SAIB; heating and stirring at 65°C until dissolved; then performing sterile filtration to obtain the pharmaceutical composition (as an in-situ gel formulation).

### Example 5

Use of a pharmaceutical composition in the preparation of a drug for enhancing analgesic effect is provided. Raw material components and amount of the pharmaceutical composition are shown in Table 1.

A preparation method of the pharmaceutical composition in Example 5 included the following steps:
mixing bupivacaine, L-menthol, celecoxib, glycerol formal and SAIB; heating and stirring at 65°C until dissolved; then performing sterile filtration to obtain the pharmaceutical composition (as an in-situ gel formulation).

### Example 6

Use of a pharmaceutical composition in the preparation of a drug for enhancing analgesic effect is provided. Raw material components and amount of the pharmaceutical composition are shown in Table 1.

A preparation method of the pharmaceutical composition in Example 6 included the following steps:
mixing bupivacaine, L-menthol, glycerol formal and poly(orthoester); heating and stirring at 65°C until dissolved; then performing sterile filtration to obtain the pharmaceutical composition (as an in-situ gel formulation).

### Example 7

Use of a pharmaceutical composition in the preparation of a drug for enhancing analgesic effect is provided. Raw material components and amount of the pharmaceutical composition are shown in Table 1.

A preparation method of the pharmaceutical composition in Example 7 included the following steps:
mixing bupivacaine, L-menthol, glycerol formal and poly(orthoester); heating and stirring at 65°C until dissolved; then performing sterile filtration to obtain the pharmaceutical composition (as an in-situ gel formulation).

### Example 8

Use of a pharmaceutical composition in the preparation of a drug for enhancing analgesic effect is provided. Raw material components and amount of the pharmaceutical composition are shown in Table 1.

A dosage form of the pharmaceutical composition in Example 8 was liposome, and a preparation method of the pharmaceutical composition included the following steps:
mixing DEPC and DPPG in glycerol trioctanoate to obtain solution 1; mixing bupivacaine and L-menthol in water to obtain solution 2; then mixing solution 1 and solution 2 to obtain a primary emulsion; shearing the primary emulsion to obtain a multiple emulsion; and then obtaining the pharmaceutical composition (as a liposome formulation) by replacement concentration.

### Example 9

Use of a pharmaceutical composition in the preparation of a drug for enhancing analgesic effect is provided. Raw material components and amount of the pharmaceutical composition are shown in Table 1.

A preparation method of the pharmaceutical composition in Example 9 included the following steps:
mixing bupivacaine, L-menthol, benzyl alcohol and SAIB; heating and stirring at 65°C until dissolved; and then performing sterile filtration to obtain the pharmaceutical composition (as an in-situ gel formulation).

### Comparative Example 1

The differences between Comparative Example 1 and Example 1 were that, in Comparative Example 1, an equal amount of bupivacaine was used to replace L-menthol, i.e., no L-menthol was included, and the amount of glycerol formal used was 10 parts, and no SAIB was included. The rest of Comparative Example 1 was the same as in Example 1.

### Comparative Example 2

The differences between Comparative Example 2 and Example 1 were that, in Comparative Example 2, an equal amount of L-menthol was used to replace bupivacaine, i.e., no bupivacaine was included, and the amount of glycerol formal used was 10 parts, and no SAIB was included. The rest of Comparative Example 2 was the same as in Example 1.

### Comparative Example 3

The only difference between Comparative Example 3 and Example 1 was that, in Comparative Example 3, an equal amount of bupivacaine was used to replace L-menthol, i.e., no terpene drug was included. The rest of Comparative Example 3 was the same as in Example 1.

### Comparative Example 4

The only difference between Comparative Example 4 and Example 1 was that, in Comparative Example 4, an equal amount of L-menthol was used to replace bupivacaine, i.e., no analgesic drug was included. The rest of Comparative Example 4 was the same as in Example 1.

### Comparative Example 5

The differences between Comparative Example 5 and Examples 1-3 were that, in Comparative Example 5, no delivery carrier was included, and the amount of glycerol formal used was 10 parts. The rest of Comparative Example 5 was the same as in Example 1, Example 2 or Example 3.

### Comparative Example 6

The only difference between Comparative Example 6 and Example 6 was that the amount of L-menthol used in Comparative Example 6 was 5 parts, i.e., the amount of L-menthol used was increased. The rest of Comparative Example 6 was the same as in Example 6.

### Comparative Example 7

The only difference between Comparative Example 7 and Example 4 was that no L-menthol was included in Comparative Example 7. The rest of Comparative Example 7 was the same as in Example 4.

### Comparative Example 8

Comparative Example 8 used the existing analgesic drug, Zynrelef.

### Performance test

### 1. Release test of bupivacaine

The pharmacokinetics of bupivacaine in rats was investigated to determine the plasma concentration of bupivacaine and thereby estimating whether L-menthol had an effect on in vivo release of bupivacaine.

The specific process for researching the pharmacokinetics in rats was as follows:
Male SD rats weighing approximately 200-250 g were selected, and the pharmaceutical compositions of Example 2-3, Comparative Example 1, Comparative Example 3, and Comparative Example 5 were respectively administered subcutaneously into the back of the SD rats at a dose of 30.0 mg/kg. Blood samples of approximately 0.5 mL were collected from the SD rats at 0.25, 0.5, 1, 2, 3, 6, 12, 24, 48, 72, and 96 hours respectively after administration, and placed in EDTA-2K + anticoagulant blood collection tubes. The whole blood was centrifuged at 8,000 rpm for 5 minutes to collect plasma, and then the concentration of bupivacaine in the plasma samples of the SD rats was determined by liquid chromatography-tandem mass spectrometry (LC-MS/MS).

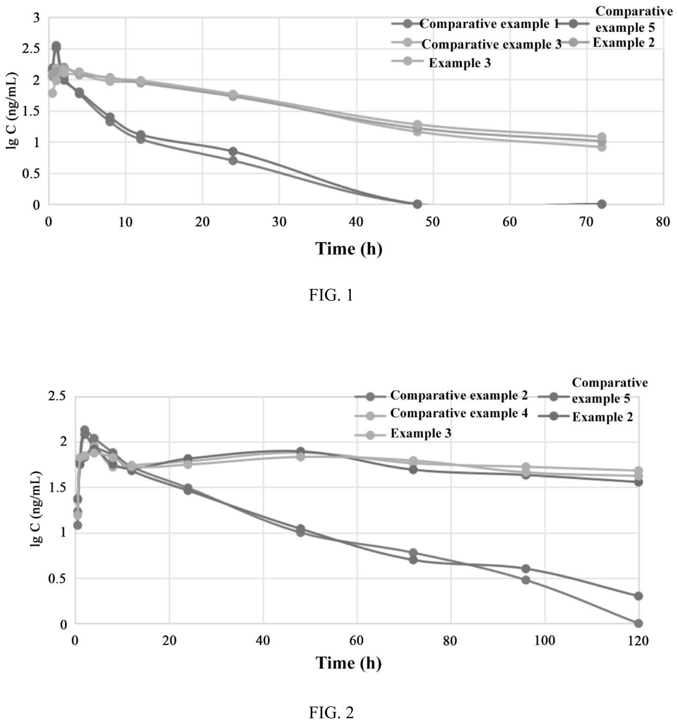

Logarithmic curves of plasma concentration of the local anesthetic bupivacaine in rats after administration of the pharmaceutical compositions of Examples 2-3, Comparative Example 1, Comparative Example 3, and Comparative Example 5 are shown in Fig. 1, where lgC on the vertical axis represents the logarithm of plasma concentration.

It can be seen from Fig. 1 that, the time to peak plasma concentration of bupivacaine in Comparative Example 1 and Comparative Example 5 is approximately 1 hour, respectively, and no sustained-release characteristic is observed, indicating that the addition of the terpene drug L-menthol cannot change the release of bupivacaine. The formulations of Comparative Example 3 and Examples 2 and 3 all contain bupivacaine and a sustained-release carrier. In Comparative Example 3 and Examples 2 and 3, the plasma concentrations of bupivacaine exhibit sustained-release characteristic. Although L-menthol is added in Examples 2 and 3, the release characteristics of bupivacaine in the rats are not changed in both sustained-release systems whether L-menthol is added or not. This demonstrates that the addition of terpene drug did not change the in vivo release of the analgesic drug.

### 2. Plasma concentration test of L-menthol

The plasma concentrations of menthol in the rats after administration of the pharmaceutical compositions of Examples 2-3, Comparative Example 2, Comparative Example 4, and Comparative Example 5 were determined using the same method as described above for the plasma concentration test of bupivacaine.

Logarithmic curves of plasma concentration of the terpene drug L-menthol in rats after administration of the pharmaceutical compositions of Examples 2-3, Comparative Example 2, Comparative Example 4 and Comparative Example 5 are shown in Fig. 2, where lgC on the vertical axis represents the logarithm of plasma concentration.

It can be seen from Fig. 2 that there is no sustained-release characteristic of plasma concentration of L-menthol in rats after administration of the pharmaceutical compositions of Comparative Example 2 and Comparative Example 5. Comparative Example 4 and Example 2 and Example 3 all contain L-menthol and a delivery carrier, and their plasma concentrations in vivo have sustained-release characteristics, which can last for more than 120 hours.

### 3. Analgesic effect test

### (1) Research on analgesic effect of drugs in SD rats

To investigate the analgesic effect of the pharmaceutical composition of the present disclosure in rats, a research on postoperative plantar analgesic effects was conducted on the pharmaceutical compositions of Example 2 and Comparative Examples 3-4 in rats, and the specific process was as follows.

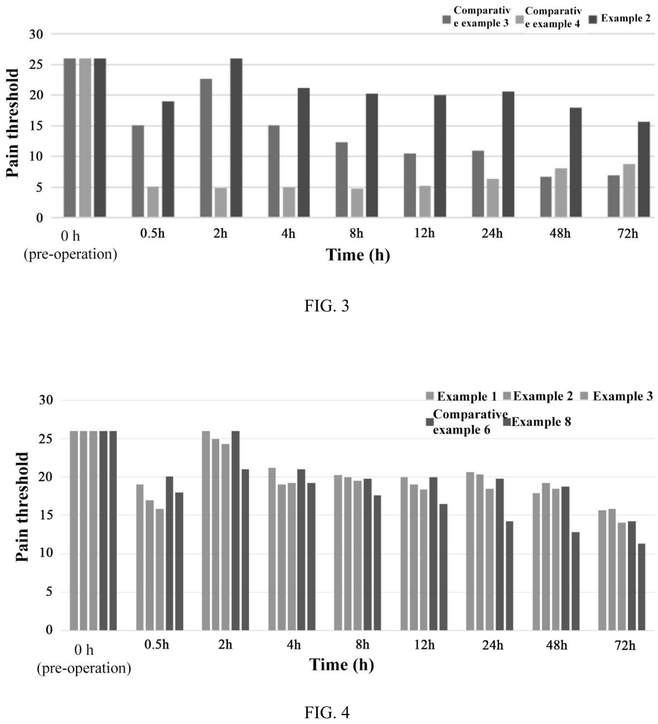

Specific pathogen-free (SPF) male SD rats aged 5-6 weeks were selected. After anesthesia with isoflurane, a 1.0 cm incision was made on the planta pedis, and the muscle was cut open without affecting its start, stop and attachment. After suturing, the pharmaceutical composition was administered by infiltration injection adjacent to the incision. Each SD rat was administered the pharmaceutical composition of Example 2, Comparative Example 3-4 at a dose of 15 mg/kg. Von Frey mechanical hyperalgesia tests were performed on SD rats before surgery and administration (0 h) and at 0.5, 2, 4, 8, 12, 24, 48, and 72 hours after administration, using nine Von Frey filaments with forces equivalent to 1, 1.4, 2, 4, 6, 8, 10, 15, and 26 g respectively. The "up-and-down" method was employed to detect and record the test results and calculate the pain threshold. The analgesic effects in rats after administration of the pharmaceutical compositions of Example 2 and Comparative Examples 3-4 are shown in Fig. 3. Smaller vertical axis data indicate more obvious pain and lower drug efficacy.

From the experimental results in Fig. 3, it can be seen that the Example 2 of the present disclosure uses a pharmaceutical composition including a combination of bupivacaine and L-menthol, which exert good analgesic effect as early as 0.5 hours after administration. The maximum analgesic threshold comparable to the preoperative level is achieved at 2 hours after administration, and an analgesic effect substantially comparable to that at 0.5 hours after administration is still maintained even at 72 hours after administration. This indicates that the pharmaceutical composition system including a combination of bupivacaine and L-menthol according to the present disclosure provides good analgesic effect and can prolong the duration of analgesic effect. That is, the pharmaceutical composition of the present disclosure has a long-acting analgesic effect. In addition, the pharmaceutical composition exhibits rapid onset of analgesia.

Comparative Example 3 does not contain L-menthol and is a sustained-release system of single bupivacaine formulation, which has certain analgesic effect and long-acting analgesic effect. However, the analgesic effect and long-acting analgesic effect of Comparative Example 3 are both inferior to those of Example 2, especially the long-acting analgesic effect. Comparative Example 4 does not contain bupivacaine and is a sustained-release of single L-menthol formulation, showing no significant analgesic effect. Only slight increase in pain threshold in Comparative Example 4 is observed at 24 h, 48 h, and 72 h, resulting in a slightly improved analgesic effect. This may be attributed to, on the one hand, the sustained-release effect of the L-menthol in Comparative Example 4, and on the other hand, the fast metabolism of rats and the gradual recovery and healing of the wounds over time. This demonstrates that the combination of bupivacaine and L-menthol can endow the pharmaceutical composition with favorable analgesic effect, enhanced long-acting analgesic effect, and a shortened onset time of analgesia.

The same test method as the above for analgesic effect after plantar surgery in rats was used to evaluate the analgesic effect of the pharmaceutical compositions in Examples 1-3, Example 8, and Comparative Example 6, and the analgesic effect of the pharmaceutical compositions in Examples 1-3, Example 8, and Comparative Example 6 in rats after administration are shown in Fig. 4.

It can be seen from Fig. 4 that Examples 1-3 of the present disclosure use a sustained-release composition including a combination of bupivacaine and L-menthol with different delivery carriers. The analgesic effects and durations of analgesic effect of Examples 1-3 are comparable, all exerting favorable analgesic effect and long-acting analgesic effect.

Example 8 is a liposome formulation using phospholipid system as a delivery carrier, which has good analgesic effect, but the duration of drug efficacy is inferior to Examples 1-3.

The amount of L-menthol in Comparative Example 6 is 5 parts, which means that increasing the amount of L-menthol has a good long-acting analgesic effect. However, during the experiment, slight redness and swelling appear at the administration site, indicating that a higher proportion of L-menthol will produce local irritation, reducing safety and patient compliance.

In addition, based on the potential adverse effects such as redness and swelling at the administration site of the rats, etc., the same administration method as described above was used to monitor whether there was redness and swelling at the administration site after administration of the pharmaceutical compositions in Examples 6 and 7.

The conditions of the administration site after administration of the pharmaceutical compositions in Examples 6-7 and Comparative Example 6 are shown in Table 2.

**Table 2: Conditions of administration site after administration of pharmaceutical compositions in Examples 6-7 and Comparative Example 6**

| Groups | Usage amount of bupivacaine and L-menthol | Whether redness and swelling occur at the administration site | Other adverse effects |
|---|---|---|---|
| Comparative Example 6 | 1 part of bupivacaine and 5 parts of L-menthol | Yes | No |
| Example 6 | 1 part of bupivacaine and 1 part of L-menthol | No | No |
| Example 7 | 1 part of bupivacaine and 0.5 parts of L-menthol | No | No |

It can be seen from Table 2 that there are redness and swelling at the administration site in Comparative Example 6. In Examples 6 and 7, the usage amount of L-menthol is reduced, and there are no redness, swelling, and other adverse effects at the administration site of the rats. Therefore, a weight ratio of bupivacaine to L-menthol should not be less than 1:1.

### (2) Research of drug analgesic effect in Bama miniature pigs

To evaluate the analgesic effect of the pharmaceutical composition of the present disclosure in Bama miniature pigs, an analgesia test was conducted on the postoperative acute pain model in Bama miniature pigs using the pharmaceutical compositions in Examples 4-5 and Comparative Example 7 and the existing analgesic drug Zynrelef in Comparative Example 8.

Specific pathogen-free (SPF) Bama miniature pigs were selected, and anesthesia was induced by intravenous injection of propofol injection (2-4 mg/kg body weight) through the femoral vein. A longitudinal incision of 7 cm was made at 3 cm to the left of the spinal line in the lower back using a scalpel. The skin and fascia layers were bluntly dissected with hemostatic forceps. The Bama miniature pigs were then administered, at a dose of 10 mg/kg, with pharmaceutical compositions in Example 4, Example 5, and Comparative Example 7 and the existing analgesic drug Zynrelef in Comparative Example 8 respectively. After suturing, the incision was disinfected with iodophor to prevent infection. Von Frey mechanical hyperalgesia test were performed on the Bama miniature pigs before surgery (0 h) and at 1 h, 3 h, 5 h, 24 h (postoperative day 1), 48 h (postoperative day 2), 72 h (postoperative day 3), and 120 h (postoperative day 5) after administration, using nine Von Frey filaments with forces equivalent to 1.4, 2, 4, 6, 8, 10, 15, 26 and 60 g, respectively. The "up-and-down" method was employed to detect and record the test results and calculate the pain threshold.

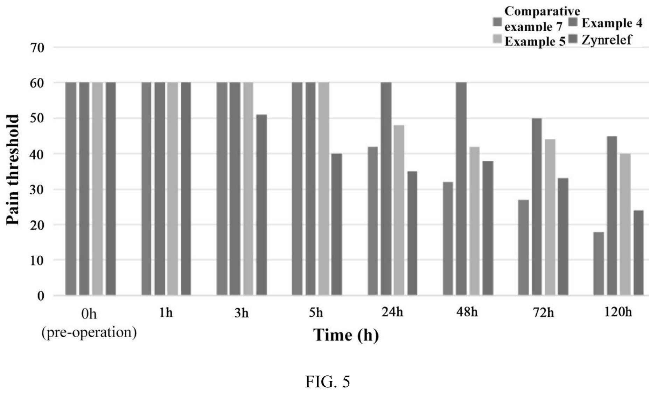

The analgesic effects of the pharmaceutical compositions in Example 4, Example 5, and Comparative Example 7 and the analgesic drug Zynrelef in Comparative Example 8 in Bama miniature pigs after administration are shown in Fig. 5. Smaller vertical axis data indicate more obvious pain and lower drug efficacy.

It can be seen from Fig. 5 that the pharmaceutical compositions in Examples 4 and 5 of the present disclosure employ a drug system involving local anesthetic bupivacaine, nonsteroidal anti-inflammatory drug meloxicam, and terpene drug L-menthol, can reach an analgesic threshold comparable to the preoperative level at 1 hour after administration, and maintain analgesic effect for up to 120 hours, demonstrating both favorable postoperative analgesic effects and long-acting analgesic properties effect. In particular, Example 4 exhibits a pain threshold equivalent to the preoperative level even at 48 hours after administration, indicating excellent analgesic effect and long-acting efficacy.

Although no L-menthol is added In Comparative Example 7, Comparative Example 7 has good postoperative analgesic effect within 24 hours. However, with prolonged time, the analgesic effect of Comparative Example 7 is significantly inferior to that of Examples 4-5, indicating poor long-acting analgesic effect.

In addition, the drug-loading capacity of local anesthetic and nonsteroidal anti-inflammatory drug in Example 5 is half of that in Example 4 and half of that in Comparative Example 7. However, due to the presence of L-menthol in Example 5, even when the drug-loading capacity of local anesthetic and nonsteroidal anti-inflammatory drug is significantly reduced, the analgesic threshold of Example 5 at 120 hours after administration is still significantly higher than that of Comparative Example 7, indicating that its long-acting analgesic effect is significantly superior to that of Comparative Example 7. This demonstrates that the terpene drug can effectively prolong the time for analgesic drugs to exert good analgesic effect. That is, when an analgesic drug and a terpene drug are used in combination, the pharmaceutical composition can have good analgesic effect and long-acting analgesic effect.

Starting from 3 hours after administration, the drug Zynrelef in Comparative Example 8 begin to show an analgesic effect inferior to that of Example 4 and Example 5. With prolonged time, the analgesic effect of Zynrelef significantly decreases, and the analgesic effect is significantly inferior to that of Example 4 and Example 5. This demonstrates that the long-acting analgesic effect of the pharmaceutical composition prepared by the present disclosure is significantly superior to that of the marketed drug Zynrelef.

The pharmaceutical compositions in Examples 6, 7, and 9 of the present disclosure also exhibit good long-acting analgesic effects.

In summary, the analgesic drug and the terpene drug of the present disclosure have a synergistic effect. Without changing the plasma concentration of an analgesic drug in vivo, the terpene drug can increase the drug-loading capacity of the analgesic drug, enabling the pharmaceutical composition to have good analgesic effect and long-acting analgesic effect. The postoperative analgesic duration for Bama miniature pigs can be extended to 120 hours, which is significantly superior to the 72 hours achieved by existing marketed drugs, and a rapid onset of analgesia is achieved.

The above examples are merely intended to illustrate the technical scheme of the present disclosure and do not limit the scope of protection thereof. Although the present disclosure has been described in detail with reference to the preferred examples, those having ordinary skills in the art should understand that modifications or equivalent substitutions can be made to the technical scheme of the present disclosure without departing from the essence and scope of the technical scheme of the present disclosure.

## Claims

1. A pharmaceutical composition for use in the treatment of pain, **characterized in that** the pharmaceutical composition comprises an analgesic drug and a terpene drug;
the analgesic drug comprises at least one of a local anesthetic and a nonsteroidal anti-inflammatory drug; and
the terpene drug comprises at least one of menthol, camphor, linalool, and eucalyptol.

2. The pharmaceutical composition for use of claim 1, **characterized in that** the local anesthetic comprises at least one of bupivacaine and ropivacaine.

3. The pharmaceutical composition for use of claim 1, **characterized in that** the nonsteroidal anti-inflammatory drug comprises at least one of meloxicam and celecoxib.

4. The pharmaceutical composition for use of claim 1, **characterized in that** the pharmaceutical composition further comprises at least one of a delivery carrier and a solvent.

5. The pharmaceutical composition for use of claim 4, **characterized in that** the solvent comprises at least one of alcohols, dimethyl sulfoxide, ethyl lactate, glycerol formal, N-methylpyrrolidone, glycerol triacetate, benzyl benzoate, dichloromethane, water, and glycerol trioctanoate.

6. The pharmaceutical composition for use of claim 4, **characterized in that** the delivery carrier comprises at least one of a saccharide and an esterified form thereof, a phospholipid, a polyester, and an oil.

7. The pharmaceutical composition for use of claim 6, **characterized in that** the saccharide and an esterified form thereof comprise at least one of sucrose, chitosan, sucrose acetate isobutyrate, sucrose octaacetate, and sucrose monoacetate monoisobutyrate; and/or, the phospholipid comprises at least one of a natural phospholipid and a synthetic phospholipid; and/or, the oil comprises at least one of castor oil, soybean oil, olive oil, palm oil, and sesame oil; and/or, the polyester comprises at least one of polylactic acid, poly(lactic-co-glycolic acid), polyglycolic acid, polydioxanone, polycaprolactone, poly(L-lactide-co-caprolactone), and poly(orthoester).

8. The pharmaceutical composition for use of claim 7, **characterized in that** the natural phospholipid comprises at least one of soybean phosphatidylcholine, egg yolk phospholipid, rapeseed phospholipid, and sunflower phospholipid.

9. The pharmaceutical composition for use of claim 7, **characterized in that** the synthetic phospholipid comprises at least one of dierucoyl phosphatidylcholine, dioleoyl phosphatidylcholine, palmitoyl oleoyl phosphatidylcholine, distearoyl phosphatidylcholine, dipalmitoyl phosphatidylglycerol, and distearoyl phosphatidylglycerol.

10. The pharmaceutical composition for use of claim 7, **characterized in that** the poly(orthoester) is selected from a non-thermoplastic poly(orthoester) having the following structure: 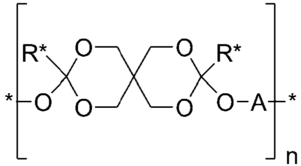
wherein R* is selected from any one of methyl, ethyl, propyl, or butyl; n is an integer from 5 to 400; and A is selected from any one of R¹ or R³;
R¹ is 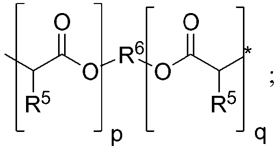 R⁵ is selected from any one of H or C₁₋₄ alkyl; R⁶ is 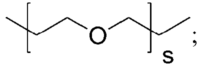 s is an integer from 0 to 10; and p and q are each independently selected from an integer from 1 to 20;
R³ is 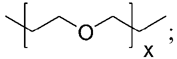 and x is an integer from 1 to 5.

11. The pharmaceutical composition for use of claim 4, **characterized in that** the pharmaceutical composition further comprises a delivery carrier and a solvent; and in parts by weight, the pharmaceutical composition comprises 0.01-22 parts of an analgesic drug, greater than or equal to 0.01 parts and less than 5 parts of a terpene drug, 1-95 parts of a solvent, and 0.5-65 parts of a delivery carrier.

12. The pharmaceutical composition for use of claim 10, **characterized in that** a mass ratio of the local anesthetic to the terpene drug is 1 to 50: 1.

13. The pharmaceutical composition for use of any one of claims 1 to 12, **characterized in that** a formulation form of the pharmaceutical composition comprises any one of gel formulation or liposome.

14. The pharmaceutical composition for use of any one of claims 1 to 13, **characterized in that** a preparation method of the pharmaceutical composition comprises the step of:
mixing raw material components to obtain the pharmaceutical composition.

15. Use of the pharmaceutical composition for use of any one of claims 1 to 14 for relieving pain or enhancing analgesic effect.
